# FASCICULE DE BREVET EUROPEEN

(11) **EP 0 603 078 B1**
(45) Date de publication et mention de la délivrance du brevet: **30.07.1997**
(21) Numéro de dépôt: 93403066.9
(22) Date de dépôt: 17.12.1993
(51) Int. Cl.: A61K 7/08, A61K 7/06, A61K 7/48, A61K 7/50

(54) **Compositions de conditionnement des matières kératiniques à base d'alkylpolyglycosides et leur utilisation pour le lavage et le conditionnement des cheveux**
Mittel zum Konditionieren von Keratinmaterial auf Basis von Alkylpolyglycosiden und ihre Verwendung zum Waschen und Konditionieren von menschlichen Haaren
Conditioning compositions for keratinous material based on alkylpolyglycosides and their use for washing and conditioning hair

(30) Priorité: 18.12.1992 FR 9215676
(43) Date de publication de la demande: 22.06.1994
(73) Titulaire: L'OREAL, 75008 Paris (FR)
(72) Inventeur: Cauwet, Danièle, F-75011 Paris (FR); Dubief, Claude, F-78150 Le Chesnay (FR)
(74) Mandataire: Casalonga, Axel

(56) Documents cités:
- EP-A- 0 203 750
- EP-A- 0 269 243
- EP-A- 0 337 354
- EP-A- 0 521 748
- EP-A- 0 531 650
- DATABASE WPI Week 9225, Derwent Publications Ltd., London, GB; AN 92-204464 & JP-A-4 134 019 (LION CORP) 7 Mai 1992

## Description

La présente invention est relative à des compositions de conditionnement des matières kératiniques et plus particulièrement des cheveux humains ou de la peau, à base d'alkylpolyglycosides et d'un polymère amphotère dérivé de diallyldialkylammonium et d'un monomère acide choisi parmi l'acide acrylique ou méthacrylique et aux procédés de traitement des matières kératiniques mettant en oeuvre de telles compositions.

L'invention concerne plus particulièrement des compositions de lavage ou shampooings ainsi que des compositions après-shampooings dont l'application est généralement suivie d'un rinçage.

Les compositions de lavage des cheveux ou shampooings sont généralement formulées à partir d'agents tensio-actifs anioniques ou non-ioniques ou leurs mélanges, en présence éventuellement d'agents tensio-actifs amphotères.

Utilisés seuls, ces agents tensio-actifs anioniques ou non-ioniques ne conduisent pas à de bonnes propriétés cosmétiques et, notamment, le démêlage des cheveux humides est difficile et les cheveux sont rêches.

On a proposé de rajouter à ces compositions de lavage des polymères cationiques et/ou des silicones, afin d'améliorer leurs propriétés cosmétiques.

On a cependant constaté que dans un milieu anionique, la performance des polymères cationiques n'est pas optimale en raison de l'interaction ionique entre le polymère cationique et l'agent tensioactif anionique et on se trouve parfois en présence de problèmes de stabilité de la composition.

L'introduction de silicone dans de telles solutions à base d'agents tensio-actifs anioniques ou non-ioniques pose également des problèmes, notamment au niveau de la formulation, ce qui entraîne également des problèmes au niveau de la stabilité de telles compositions.

On a également proposé d'incorporer dans des compositions de lavage contenant des alkylpolyglycosides, des polymères cationiques. Ces compositions détergentes présentent cependant des problèmes d'élimination au rinçage, ce qui entraîne un manque de douceur des cheveux séchés et un démêlage non satisfaisant des cheveux humides.

Dans les compositions après-shampooings, on a déjà proposé dans le passé d'utiliser des agents tensio-actifs cationiques et/ou des polymères cationiques qui présentent cependant l'inconvénient de s'accumuler sur les cheveux au cours des applications successives, conduisant ainsi à un alourdissement de la chevelure et lui conférant un toucher désagréable.

La demanderesse a découvert, ce qui fait l'objet de l'invention, une composition permettant de conditionner les cheveux pouvant être utilisée pour le lavage ou comme après-shampooing et contenant un alkylpolyglycoside et un polymère amphotère dérivé de diallyldialkylammonium et d'un monomère acide choisi parmi l'acide acrylique ou méthacrylique. Cette composition ne contient pas de silicone comme agent de conditionnement ou de savon comme agent de lavage.

Les compositions conformes à l'invention présentent, notamment, la caractéristique d'être particulièrement stables par rapport à des compositions contenant en plus comme agent de conditionnement une silicone.

Les compositions conformes à l'invention présentent également l'avantage de conférer aux cheveux traités de la douceur, de la légèreté et permettent aux cheveux de sécher plus rapidement lorsque la composition ne contient pas de savon. Enfin, ces compositions s'éliminent plus facilement au rinçage et se démêlent plus facilement lorsque les cheveux sont mouillés.

L'invention a donc pour objet une composition de conditionnement des matières kératiniques, notamment des cheveux, utilisable comme shampooing ou comme après-shampooing en vue de conditionner les matières traitées.

L'invention a également pour objet un procédé de lavage des matières kératiniques et en particulier des cheveux et de la peau mettant en oeuvre une telle composition.

Un autre objet de l'invention est constitué par un procédé de traitement après shampooing, mettant en oeuvre la composition définie ci-dessus, l'application étant suivie d'un rinçage.

D'autres objets de l'invention apparaîtront à la lecture de la description et des exemples qui suivent.

La composition de conditionnement des matières kératiniques et plus particulièrement des cheveux humains, est essentiellement caractérisée par le fait qu'elle contient en milieu aqueux de 0,1 à 10% en poids par rapport au poids total de la composition d'un copolymère constitué de 70 à 90% en poids d'unités de diallyldialkylammonium et de 30 à 10% en poids d'un monomère acide choisi parmi l'acide acrylique ou méthacrylique et de 0,1 à 40% en poids par rapport au poids total de la composition, d'un alkylpolyglycoside répondant à la formule :

R(C₆H₁₀O₅)ₓ H (I)

ou correspondant encore à la formule développée (II) : 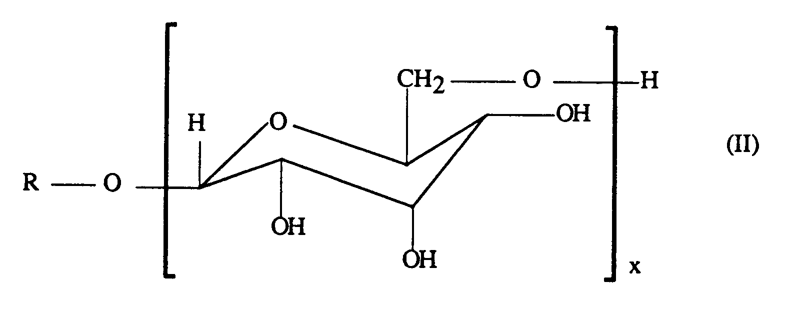 dans laquelle :
R désigne un radical ou un mélange de radicaux alkyle ou alcényle à chaîne droite ou ramifiée en C₈-C₂₄ et x est un nombre de 1 à 15, cette composition ne contenant ni silicone à titre d'agent de conditionnement, ni savon comme agent détergent.

Les groupements alkyle du monomère diallyldialkylammonium sont choisis indépendamment l'un de l'autre parmi les groupements alkyle ayant 1 à 18 atomes de carbone et représentent de préférence un radical méthyle ou éthyle. Le poids moléculaire du polymère amphotère défini ci-dessus est compris entre environ 50.000 et 10.000.000 déterminé par chromatographie par perméation de gel et de préférence compris entre 200.000 et 5.000.000. De tels polymères sont décrits plus particulièrement dans la demande EP-A-0269243.

Parmi ces polymères, les copolymères de chlorure de diméthyldiallylammonium ou de diéthyldiallylammonium et d'acide acrylique sont particulièrement préférés.

On peut plus particulièrement citer le polymère vendu sous la dénomination MERQUAT 280 par la Société CALGON, sous la forme d'une solution aqueuse à 35% de matière active, ce polymère étant un copolymère de chlorure de diallyldiméthylammonium et d'acide acrylique dans les proportions 80/20, la viscosité au viscosimètre Brookfield LVF, module 4 à 60 tours par minute, étant comprise entre 3,5 Pa.s et 17 Pa.s, le poids moléculaire étant environ égal à 1.300.000.

Les alkylpolyglycosides sont choisis plus particulièrement parmi les produits vendus par la Société HENKEL sous la dénomination APG, tels que les produits APG 300, APG 350, APG 500, APG 550, APG 625, APG base 10-12; les produits vendus par la Société SEPPIC sous les dénominations TRITON CG 110 (ou ORAMIX CG 110) et TRITON CG 312 ou (ORAMIX NS 10); ceux vendus par la Société BASF sous la dénomination LUTENSOL GD 70.

Les alkylpolyglycosides de formule (I) ou (II) préférés, utilisés conformément à l'invention, sont l'APG 300 de la Société HENKEL et le LUTENSOL GD 70 de la Société BASF.

Lorsque les compositions conformes à l'invention sont utilisées pour le lavage des fibres kératiniques et plus particulièrement des cheveux, la concentration en alkylpolyglycoside définie ci-dessus varie de 1 à 40% en poids et plus préférentiellement de 5 à 30% en poids par rapport au poids total de la composition et celle du copolymère dérivé de diallyldialkylammonium et d'acide acrylique ou méthacrylique est de préférence comprise entre 0,5 et 5% en poids par rapport au poids total de la composition.

Les compositions de lavage ou shampooing peuvent contenir en plus de l'alkylpolyglycoside, d'autres agents co-tensio-actifs à l'exception des savons. Ces agents co-tensio-actifs autres que les savons sont connus en eux-mêmes et sont choisis parmi les agents tensio-actifs anioniques, amphotères, zwittérioniques, non-ioniques, cationiques ou leurs mélanges, ayant des propriétés détergentes.

Parmi les agents tensio-actifs anioniques, on peut citer les sels alcalins, les sels d'ammonium, les sels d'amines, les sels d'aminoalcools, les sels de magnésium des composés suivants : les alkylsulfates, alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylétherphosphates; les acylsarcosinates, les N-acyltaurates.

Le radical alkyle ou acyle de ces différents composés est généralement constitué par une chaîne carbonée comportant de 10 à 20 atomes de carbone.

On peut également utiliser des agents tensio-actifs faiblement anioniques, tels que les acides éthers carboxyliques polyoxyalkylénés, tels que ceux comportant 2 à 50 groupements oxyde d'éthylène.

Les agents tensio-actifs non-ioniques différents de ceux de formule (I) sont plus particulièrement choisis parmi les alcools ou les α-diols ou les alkylphénols ou les acides gras polyéthoxylés, polypropoxylés ou polyglycérolés, à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30.

On peut citer plus particulièrement les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4 groupements glycérol; les amines grasses polyéthoxylées ayant de préférence 2 à 30 moles d'oxyde d'éthylène; les esters d'acides gras du sorbitan oxyéthylénés ayant de préférence 2 à 30 moles d'oxyde d'éthylène; les esters d'acides gras de sucre, les esters d'acides gras du polyéthylèneglycol, les esters d'acides gras de glycols, les oxydes d'amines tels que les oxydes d'alkyl(C₁₀-C₁₄) amines ou de N-acylamidopropylmorpholine.

Les agents tensio-actifs amphotères ou zwittérioniques préférés sont les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les alkyl(C₈-C₂₀)bétaïnes, les sulfobétaines, les alkyl(C₈-C₂₀)amidoalkyl(C₁-C₆)bétaïnes ou les alkyl (C₈-C₂₀)amidoalkyl(C₁-C₆)sulfobétaïnes.

Parmi les dérivés d'amines, on peut citer les produits commercialisés sous la dénomination MIRANOL, tels que ceux décrits dans les brevets US-A-2.528.378 et 2.781.354 ou classés dans le dictionnaire CTFA, 3ème édition, 1982, sous les dénominations d'Amphocarboxyglycinates ou d'Amphocarboxypropionates.

Les agents co-tensio-actifs sont utilisés dans les compositions de lavage conformes à l'invention dans des proportions suffisantes pour conférer, en combinaison avec l'alkylpolyglycoside obligatoirement présent dans ces compositions, un caractère détergent à la composition. Ces agents co-tensio-actifs sont généralement présents dans des proportions comprises entre 0,5 et 30% en poids par rapport au poids total de la composition et en particulier entre 1 et 20% en poids.

Les compositions conformes à l'invention peuvent également contenir d'autres agents et plus particulièrement des agents renforçateurs de mousse ou "boosters" tels que les alcanolamides d'acides gras et notamment de coprah.

Lorsque les compositions selon l'invention sont utilisées comme compositions après-shampooing, leur application est généralement suivie d'un rinçage. Dans ces compositions, la concentration en alkylpolyglycoside varie de 0,1 à 10% et de préférence de 0,3 à 5% en poids par rapport au poids total de la composition et la concentration en copolymère de diallyldialkylammonium et d'acide acrylique ou méthacrylique varie de 0,1 à 10% et de préférence de 0,3 à 5% en poids par rapport au poids total de la composition. Elles peuvent contenir des agents co-tensio-actifs anioniques à l'exclusion des savons, amphotères, non-ioniques ou cationiques, définis ci-dessus, ces agents étant présents dans des proportions comprises entre environ 0,05 et 5% en poids et de préférence de 0,1 à 3% en poids par rapport au poids total de la composition;

On utilise de préférence dans les compositions de conditionnement appliquées après lavage sur des matières kératiniques, les agents cotensio-actifs non-ioniques ou cationiques.

Parmi les agents co-tensio-actifs non-ioniques, on utilise de préférence les esters d'acides gras de sucres et parmi les co-tensioactifs cationiques, on utilise de préférence les chlorures d'alkyl (C₁₆-C₂₂)triméthylammonium et les sels d'ammonium quaternaires tels que le produit référencé QUATERNIUM 27 dans le CTFA, 4ème édition, vendu sous la dénomination REWOQUAT 75 PG par la Société REWO.

Les compositions conformes à l'invention peuvent également contenir les adjuvants habituellement utilisés dans les compositions cosmétiques et notamment les compositions de shampooing ou de conditionnement des cheveux.

On peut citer à cet effet les agents épaississants, des conservateurs, des parfums, éventuellement des corps gras tels que des alcools gras, des huiles ou des cires hydrocarbonées.

Dans une forme de réalisation particulière, on peut utiliser, notamment dans des après-shampooings conformes à l'invention, un mélange d'alkylpolyglycoside et d'alcool cétyl stéarylique tel que le produit commercialisé sous la dénomination MONTANOL 68.

Il est également possible d'additionner à ces compositions de conditionnement ou de lavage, des huiles synthétiques organiques telles que les isoparaffines comme plus particulièrement le produit vendu sous la dénomination ARLAMOL HD par la Société ICI.

Les compositions conformes à l'invention peuvent être utilisées sous des formes diverses, telles que de liquide, de liquide épaissi, de gel, de crème, et éventuellement être conditionnées en aérosol et être dispensées sous forme de mousse.

Le procédé de lavage et/ou de conditionnement des matières kératiniques et en particulier des cheveux, conforme à l'invention, consiste à appliquer sur ces matières au moins une composition telle que définie ci-dessus, cette application étant suivie d'une étape de rinçage à l'eau.

Les compositions de lavage peuvent être utilisées comme shampooings mais également comme gel-douche pour le lavage des cheveux et de la peau, auquel cas ils sont appliqués sur la peau et les cheveux humides qui sont rincés après application.

Lorsque les compositions sont utilisées pour le conditionnement des cheveux, elles sont appliquées sur les cheveux humides lavés au préalable avec un shampooing classique ou conforme à l'invention. Après un temps de pose de 1 à 10 minutes, on rince les cheveux à l'eau. On constate que les cheveux humides se démêlent bien, qu'ils sèchent rapidement, qu'ils sont légers même après plusieurs traitements.

Les exemples suivants sont destinés à illustrer l'invention sans pour autant présenter un caractère limitatif.

### EXEMPLES

### EXEMPLE 1

On prépare un shampooing de composition suivante :

| | |
|---|---|
| - Alkyl(C₉-C₁₀-C₁₁/20-40-40)polyglycoside (1,4), vendu à 50% de MA sous la dénomination APG 300 par la Société HENKEL | 18 g MA |
| - Copolymère de chlorure de diallyldiméthylammonium et d'acide acrylique, vendu sous la dénomination MERQUAT 280 par la Société CALGON à 35% de MA | 0,6 g MA |
| - Heptaméthylnonane vendu sous la dénomination ARLAMOL HD par la Société ICI | 1,8 g |
| - Ethers d'hexadécanediol (3 moles) et de polyéthylèneglycol (60 moles d'oxyde d'éthylène) | 2,5 g |
| - Conservateur qs | |
| - Acide chlorhydrique qs pH=7 | |
| - Eau | qsp 100 g |

### EXEMPLE 2

On prépare un shampooing de composition suivante :

| | |
|---|---|
| - Alkyl(C₉-C₁₀-C₁₁/20-40-40)polyglycoside (1,4), vendu à 50% de MA sous la dénomination APG 300 par la Société HENKEL | 12 g MA |
| - Uréthanne polyéther vendu sous la dénomination DAPRAL T212 par la Société AKZO | 1,5 g |
| - Copolymère de chlorure de diallyldiméthylammonium et d'acide acrylique, vendu sous la dénomination MERQUAT 280 par la Société CALGON à 35% de MA | 2,5 g MA |
| - Ethers d'hexadécanediol (3 moles) et de polyéthylèneglycol (60 moles d'oxyde d'éthylène) | 2,5 g |
| - Conservateur qs | |
| - Triéthanolamine qs pH=6,7 | |
| - Eau | qsp 100 g |

### EXEMPLE 3

On prépare un shampooing de composition suivante :

| | |
|---|---|
| - Alkyl(C₉-C₁₀-C₁₁/20-40-40)polyglycoside (1,4), vendu à 50% de MA sous la dénomination APG 300 par la Société HENKEL | 5 g MA |
| - Copolymère de chlorure de diallyldiméthylammonium et d'acide acrylique, vendu sous la dénomination MERQUAT 280 par la Société CALGON à 35% de MA | 0,25 g MA |
| - Tensio-actif amphotère dénommé "Cocoamphocarboxyglycinate" dans le dictionnaire CTFA, 3ène édition, 1982, vendu à la concentration de 38% de MA sous la dénomination MIRANOL C2M conc. par la Société MIRANOL | 5 g MA |
| - Dérivé de dioléate de polyéthylèneglycol (55 moles d'oxyde d'éthylène) et de propylèneglycol, vendu sous la dénomination ANTIL 141 Liquide par la Société GOLDSCHMIDT à 43,6% de MA | 1,5 g MA |
| - Conservateur qs | |
| - Acide chlorhydrique qs pH=7,6 | |
| - Eau | qsp 100 g |

### EXEMPLE 4

On prépare un shampooing de composition suivante :

| | |
|---|---|
| - Alkyl(C₉-C₁₀-C₁₁/20-40-40)polyglycoside (1,4), vendu à 50% de MA sous la dénomination APG 300 par la Société HENKEL | 20 g MA |
| - Copolymère de chlorure de diallyldiméthylammonium et d'acide acrylique, vendu sous la dénomination MERQUAT 280 par la Société CALGON à 35% de MA | 4,5 g MA |
| - Ethers d'hexadécanediol (3 moles) et de polyéthylèneglycol (60 moles d'oxyde d'éthylène) | 2,5 g |
| - Monoisopropanolamide d'acide de coprah | 2,5 g |
| - Conservateur qs | |
| - Triéthanolamine qs pH=6 | |
| - Eau | qsp 100 g |

### EXEMPLE 5

On prépare un après-shampooing de composition suivante :

| | |
|---|---|
| - Alkyl(C₉-C₁₀-C₁₁/20-40-40)polyglycoside (1,4), vendu à 50% de MA sous la dénomination APG 300 par la Société HENKEL | 2 g MA |
| - Copolymère de chlorure de diallyldiméthylammonium et d'acide acrylique, vendu sous la dénomination MERQUAT 280 par la Société CALGON à 35% de MA | 3 g MA |
| - Mélange (80/20) d'alcool cétylstéarylique et d'alcool cétylstéarylique oxyéthyléné à 33 moles d'oxyde d'éthylène, vendu sous la dénomination SINNOWAX AO par la Société HENKEL | 2 g |
| - Alcool cétylique | 1 g |
| - Alcool stéarylique | 1 g |
| - Hydroxyéthylcellulose vendue sous la dénomination NATROSOL 250 HHR par la Société AQUALON | 1 g |
| - Conservateur qs | |
| - Triéthanolamine qs pH=5 | |
| - Eau | qsp 100 g |

### EXEMPLE 6

On prépare un après-shampooing de composition suivante :

| | |
|---|---|
| - Mélange (23/77 en poids) de cétylstéarylglycoside et d'alcool cétylstéarylique, vendu sous la dénomination MONTANOL 68 par la Société SEPPIC | 8 g |
| - Copolymère de chlorure de diallyldiméthylammonium et d'acide acrylique, vendu sous la dénomination MERQUAT 280 par la Société CALGON à 35% de MA | 2 g MA |
| - Glycérine | 1,5 g |
| - Conservateur qs | |
| - Triéthanolamine qs pH=5 | |
| - Eau | qsp 100 g |

### EXEMPLE 7

On prépare un shampooing de composition suivante :

| | |
|---|---|
| - Alkyl(C₉-C₁₀-C₁₁/20-40-40)polyglycoside (1,4), vendu à 50% de MA sous la dénomination APG 300 par la Société HENKEL | 15 g MA |
| - Copolymère de chlorure de diallyldiméthylammonium et d'acide acrylique, vendu sous la dénomination MERQUAT 280 par la Société CALGON à 35% de MA | 1 g MA |
| - Eau | qsp 100 g |

Le pH est ajusté à 7 par de la triéthanolamine.

Cette composition utilisée comme shampooing permet de démêler facilement les cheveux.

### EXEMPLE 8

On prépare un shampooing de composition suivante :

| | |
|---|---|
| - PLANTAREN 1200 CS UP : alkylpolyglucoside dont le groupement alkyl est un mélange en C₁₂-C₁₄-C₁₆ | 5 g MA |
| Mélange de glycérides de palme POE (200 OE) et coprah (7 OE) | 2 g MA |
| - MERQUAT 280 | 0,35 g MA |
| - Eau | qsp 100 g |
| - HCl qs pH=6,5 | |

## Revendications

1. Composition de lavage et/ou de conditionnement des matières kératiniques, contenant en milieu aqueux 0,1 à 10% en poids par rapport au poids total de la composition d'un copolymère constitué de 70 à 90% en poids d'unités de diallyldialkylammonium et de 30 à 10% en poids d'un monomère acide choisi parmi les acides acrylique ou méthacrylique et 0,1 à 40% en poids par rapport au poids total de la composition d'un alkylpolyglycoside de formule (I) :
R(C₆H₁₀O₅)ₓ H (I)
ou répondant à la structure développée (II) : 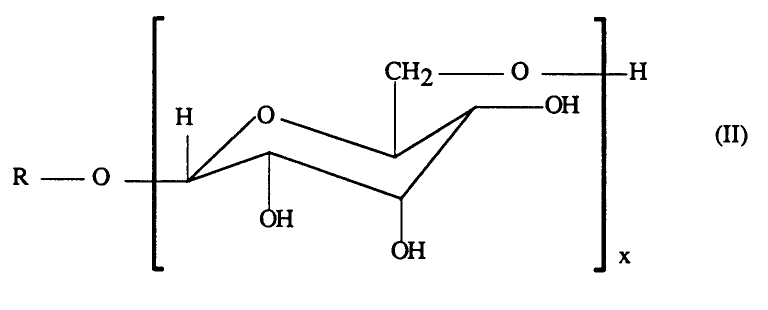 dans laquelle :
R désigne un radical ou un mélange de radicaux alkyle ou alcényle à chaîne droite ou ramifiée en C₈-C₂₄; x étant un nombre de 1 à 15, cette composition ne contenant pas de silicone comme agent de conditionnement ou de savon comme agent détergent.

2. Composition selon la revendication 1, caractérisée par le fait que le poids moléculaire du copolymère de diallyldialkylammonium et d'acide acrylique ou méthacrylique est compris entre 50.000 et 10.000.000 déterminé par chromatographie par perméation de gel.

3. Composition selon l'une des revendications 1 ou 2, caractérisée par le fait que le copolymère de diallyldialkylammonium et d'acide acrylique ou méthacrylique est choisi parmi les copolymères de chlorure de diméthyldiallylammonium ou de diéthyldiallylammonium et d'acide acrylique d'un poids moléculaire compris entre 200.000 et 5.000.000.

4. Composition selon l'une quelconque des revendications 1 à 3, caractérisée par le fait que l'alkylpolyglycoside est un alkyl(C₉-C₁₀-C₁₁)polyglycoside.

5. Composition selon l'une quelconque des revendications 1 à 4, caractérisée par le fait que la composition contient des agents cotensio-actifs autres que des savons, choisis parmi les agents tensioactifs anioniques, amphotères, zwittérioniques et non-ioniques autres que ceux de formule (I), cationiques ou leurs mélanges, ayant des propriétés détergentes.

6. Composition selon l'une quelconque des revendications 1 à 5, utilisée pour le lavage des cheveux et/ou de la peau, caractérisée par le fait que l'alkylpolyglycoside est présent dans des proportions allant de 1 à 40% et de préférence de 5 à 30% en poids par rapport au poids total de la composition et que le copolymère de diallyldialkylammonium et d'acide acrylique ou méthacrylique est présent dans des proportions comprises entre 0,5 et 5% en poids.

7. Composition selon les revendications 5 ou 6, caractérisée par le fait qu'elle contient des agents co-tensio-actifs présents dans des proportions comprises entre 0,5 et 30% en poids par rapport au poids total de la composition, et en particulier entre 1 et 20% en poids.

8. Composition selon la revendication 1 de conditionnement des cheveux, caractérisée par le fait qu'elle contient 0,1 à 10% de l'alkylpolyglycoside, de préférence 0,3 à 5% du copolymère de diallyldialkylammonium et d'acide acrylique ou méthacrylique.

9. Composition selon la revendication 8, caractérisée par le fait qu'elle contient des agents co-tensio-actifs cationiques ou non-ioniques

10. Composition selon l'une quelconque des revendications 1 à 9, caractérisée par le fait qu'elle contient des agents épaississants, des agents conservateurs, des parfums, des corps gras choisis parmi les alcools gras, les huiles ou cires hydrocarbonées.

11. Composition selon l'une quelconque des revendications 1 à 4, 8 et 9, caractérisée par le fait qu'elle contient un mélange d'alkylpolyglycoside et d'alcool cétylstéarylique.

12. Procédé de lavage des cheveux et/ou de la peau, caractérisé par le fait qu'on applique sur les cheveux et/ou la peau une composition telle que définie dans l'une quelconque des revendications 1 à 7 et 10 et qu'on procède ensuite à un rinçage à l'eau.

13. Procédé cosmétique de traitement des cheveux après leur lavage, caractérisé par le fait qu'on applique sur les cheveux humides au moins une composition telle que définie dans l'une quelconque des revendications 1 à 5, 8 et 9, et qu'après un temps de pose de 1 minute à 10 minutes, on procède à leur rinçage.

14. Utilisation d'une composition telle que définie dans l'une quelconque des revendications 1 à 7 et 10, pour le lavage des cheveux et/ou de la peau.

15. Utilisation d'une composition telle que définie dans les revendications 1 à 5, 8 et 9, pour le conditionnement des cheveux.

## Claims

1. Composition for washing and/or conditioning keratinous substances, containing, in an aqueous medium, 0.1 to 10% by weight, relative to the total weight of the composition, of a copolymer consisting of 70 to 90% by weight of diallyldialkylammonium units and 30 to 10% by weight of an acidic monomer chosen from acrylic and methacrylic acids, and 0.1 to 40% by weight, relative to the total weight of the composition, of an alkyl polyglycoside of the formula (I):
R(C₆H₁₀O₅)ₓH (I)
or corresponding to the structural formula (II): 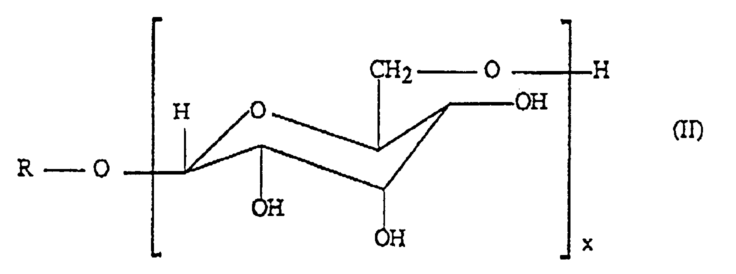 in which:
R denotes an unbranched- or branched-chain C₈-C₂₄ alkyl or alkenyl radical or a mixture of such radicals; x being a number from 1 to 15, this composition not containing silicone as a conditioning agent or soap as a detergent agent.

2. Composition according to Claim 1, characterized in that the molecular weight of the copolymer of diallyl dialkylammonium units and acrylic or methacrylic acid is between 50,000 and 10,000,000 determined by gel permeation chromatography.

3. Composition according to one of Claims 1 and 2, characterized in that the copolymer of diallyldialkylammonium units and acrylic or methacrylic acid is chosen from the copolymers of dimethyldiallylammonium or diethyl diallylammonium chloride and acrylic acid having a molecular weight of between 200,000 and 5,000,000.

4. Composition according to any one of Claims 1 to 3, characterized in that the alkyl polyglycoside is an alkyl (C₉/C₁₀/C₁₁) polyglycoside.

5. Composition according to any one of Claims 1 to 4, characterized in that the composition contains cosurfactants other than soaps, chosen from anionic, amphoteric, zwitterionic and nonionic surfactants other than those of formula (I), cationic surfactants or mixtures thereof, having detergent properties.

6. Composition according to any one of Claims 1 to 5, used for the washing of hair and/or the skin, characterized in that the alkyl polyglycoside is present in proportions ranging from 1 to 40%, and preferably from 5 to 30%, by weight relative to the total weight of the composition, and in that the copolymer of diallyldialkylammonium units and acrylic or methacrylic acid is present in proportions of between 0.5 and 5% by weight.

7. Composition according to Claim 5 or 6, characterized in that it contains cosurfactants present in proportions of between 0.5 and 30% by weight relative to the total weight of the composition, and especially between 1 and 20% by weight.

8. Hair conditioning composition according to Claim 1, characterized in that it contains 0.1 to 10 % of the alkyl polyglycoside, and preferably 0.3 to 5 % of the copolymer of diallyldialkylammonium units and acrylic or methacrylic acid.

9. Composition according to Claim 8, characterized in that it contains cationic or nonionic cosurfactants.

10. Composition according to any one of Claims 1 to 9, characterized in that it contains thickening agents, preservatives, perfumes or fats chosen from fatty alcohols, hydrocarbon waxes or oils.

11. Composition according to any one of Claims 1 to 4,8 and 9, characterized in that it contains a mixture of alkyl polyglycoside and cetyl/stearyl alcohol.

12. Process for washing hair and/or the skin , characterized in that a composition as defined in any one of Claims 1 t 7 and 10 is applied to the hair and/or skin and in that this is followed by a rinse with water.

13. Cosmetic process for treatment of hair after it is washed,characterized in that at least one composition as defined in any one of claims 1 to 5,8 and 9 is applied to the wet hair , and in that , after an exposure time of 1 minute to 10 minutes , the hair is rinsed.

14. Use of a composition as defined in any one of Claims 1 to 7 and 10, for the washing of hair and/or the skin.

15. Use of a composition as defined in Claims 1 to 5, 8 and 9, for the conditioning of hair.

## Patentansprüche

1. Zusammensetzung zum Waschen und/oder Konditionieren keratinischer Materien, enthaltend in wässrigem Medium 0,1 bis 10 Gew.%, bezogen auf das Gesamtgewicht der Zusammensetzung, eines Copolymer aus 70 bis 90 Gew.% Diallyldialkylammonium-Einheiten und aus 30 bis 10 Gew.% eines sauren Monomer, das aus Acryl- oder Methacrylsäuren ausgewählt ist, sowie enthaltend 0,1 bis 40 Gew.%, bezogen auf das Gesamtgewicht der Zusammensetzung, eines Alkylpolyglycosid der Formel (I):
R(C₆H₁₀O₅)ₓH (I)
mit der Strukturformel (II): 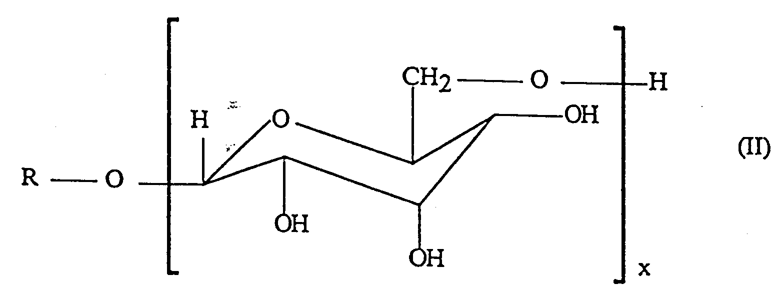 worin gilt:
R bedeutet einen Alkyl- oder Alkenylrest mit gerader oder verzweigter C₈₋₂₄-Kette oder eine Mischung dieser Reste; x ist eine Zahl von 1 bis 15,
wobei diese Zusammensetzung weder Silicon als Konditioniermittel noch Seife als Reinigungsmittel enthält.

2. Zusammensetzung gemäß Anspruch 1,
dadurch **gekennzeichnet**, daß
das Molekulargewicht des Copolymer aus Dialkyldiallylammonium-Einheiten und Acryl- oder Methacrylsäure 50000 bis 10 000 000 beträgt, bestimmt durch Gelpermeationschromatographie.

3. Zusammensetzung gemäß Anspruch 1 oder 2,
dadurch **gekennzeichnet**, daß
das Copolymer aus Diallyldialkylammonium-Einheiten und Acryl- oder Methacrylsäure aus Copolymeren aus Dimethyldiallylammonium- oder Diethyldiallylammoniumchlorid und Acrylsäure eines Molekulargewichts von 200 000 bis 5 000 000 ausgewählt ist.

4. Zusammensetzung gemäß einem der Ansprüche 1 bis 3,
dadurch **gekennzeichnet**, daß
das Alkylpolyglycosid ein (C₉-C₁₀-C₁₁)Alkylpolyglycosid ist.

5. Zusammensetzung gemäß einem der Ansprüche 1 bis 4,
dadurch **gekennzeichnet**, daß
die Zusammensetzung oberflächenaktive Zusatzmittel enthält, die sich von Seifen unterscheiden und aus anionischen, amphoteren, zwitterionischen und nicht-ionischen, die sich von denen der Formel (I) unterscheiden, und auskationischen oberflächenaktiven Mitteln oder aus deren Mischungen ausgewählt sind, wobei diese Mittel reinigende Eigenschaften aufweisen.

6. Zusammensetzung gemäß einem der Ansprüche 1 bis 5 zur Verwendung zum Waschen der Haare und/oder der Haut,
dadurch **gekennzeichnet**, daß das
Alkylpolyglycosid in Mengenanteilen von 1 bis 40 und vorzugsweise von 5 bis 30 Gew.%, bezogen auf das Gesamtgewicht der Zusammensetzung, und das Copolymer aus Diallyldialkylammonium-Einheiten und Acryl- oder Methacrylsäure in Mengenanteilen von 0,5 bis 5 Gew.% vorhanden sind.

7. Zusammensetzung gemäß Anspruch 5 oder 6,
dadurch **gekennzeichnet**, daß
sie oberflächenaktive Zusatzmittel enthält, die in Mengenanteilen von 0,5 bis 30 und insbesondere von 1 bis 20 Gew.% vorhanden sind, bezogen auf das Gesamtgewicht der Zusammensetzung.

8. Zusammensetzung gemäß Anspruch 1 zum Konditionieren der Haare,
dadurch **gekennzeichnet**, daß
sie 0,1 bis 10 % Alkylpolyglycosid und vorzugsweise 0,3 bis 5 % Copolymer aus Diallyldialkylammonium-Einheiten und Acryl- oder Methacrylsäure enthält.

9. Zusammensetzung gemäß Anspruch 8,
dadurch **gekennzeichnet**, daß
sie kationische oder nicht-ionische oberflächenaktive Zusatzmittel enthält.

10. Zusammensetzung gemäß einem der Ansprüche 1 bis 9,
dadurch **gekennzeichnet**, daß
sie Verdickungsmittel, Konservierungsmittel, Parfüm-Produkte, Fettkörper aus Fettalkoholen, Kohlenwasserstoffölen oder - wachsen enthält.

11. Zusammensetzung gemäß einem der Ansprüche 1 bis 4, 8 und 9,
dadurch **gekennzeichnet**, daß
sie eine Mischung aus Alkylpolyglycosid und Cetylstearylalkohol enthält.

12. Verfahren zum Waschen der Haare und/oder der Haut,
dadurch **gekennzeichnet**, daß
man auf die Haare und/oder die Haut eine in einem der Ansprüche 1 bis 7 und 10 definierte Zusammensetzung aufbringt und anwendet, worauf man eine Spülung mit Wasser durchführt.

13. Kosmetisches Verfahren zum Behandeln der Haare nach deren Waschung,
dadurch **gekennzeichnet**, daß
man auf die feuchten Haare mindestens eine in einem der Ansprüche 1 bis 5, 8 und 9 definierte Zusammensetzung aufbringt und anwendet, worauf man nach einer Verweilzeit von 1 bis 10 Minuten deren Spülung durchführt.

14. Verwendung einer in einem der Ansprüche 1 bis 7 und 10 definierten Zusammensetzung zum Waschen der Haare und/oder der Haut.

15. Verwendung einer in einem der Ansprüche 1 bis 5, 8 und 9 definierten Zusammensetzung zum Konditionieren der Haare.
